(19)

Europäisches
Patentamt
European
Patent Office
Office européen
des brevets

(11) **EP 2 053 401 A1**

(12) **EUROPEAN PATENT APPLICATION**

(43) Date of publication:
**29.04.2009 Bulletin 2009/18**

(51) Int Cl.:
**G01N 33/569** (2006.01)   **G01N 30/86** (2006.01)

(21) Application number: **07301485.4**

(22) Date of filing: **23.10.2007**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **I.N.R.A.**
**Institut National de la Recherche Agronomique**
**75341 Paris Cedex 07 (FR)**

(72) Inventors:
• **Loisel, Patrice**
**34170 Castelnau le Lez (FR)**

• **Hamelin, Jérôme**
**11200 Ornaissons (FR)**
• **Godon, Jean-Jacques**
**11200 Montseret (FR)**
• **Haegeman, Bart**
**11100 Narbonne (FR)**
• **Harmand, Jérôme**
**34210 La Liviniere (FR)**

(74) Representative: **Catherine, Alain et al**
**Cabinet Harlé & Phélip**
**7 rue de Madrid**
**75008 Paris (FR)**

(54) **A method for measuring the biological diversity of a sample**

(57)    This invention concerns a computer-implemented method for estimating diversity in a sample, which can apply to any sample, and in particular to a sample whom species distribution is unknown.

The method of the invention comprises steps essentially involving two components, (a) a migration of a sample comprising molecules on a surface area, whereby molecules migrate on a surface area according to their nucleotide or aminoacid sequence and (b) method steps for the diversity measurement, which are preferably performed through the working of one or several computer program(s).

EP 2 053 401 A1

**Description**

**Field of the invention**

The present invention relates to a method for measuring the biological diversity of a sample. This method is based on the characterisation of densitograms obtained from electrophoresis or chromatography performed with samples comprising nucleic acids and/or proteins.

**Background of the invention**

It is obvious that microbes play a fundamental role on earth. The ability to measure bacterial diversity is a prerequisite for the systematic study of bacterial biogeography and community assembly. It is for example central to the ecology of surface waters, the oceans and soils, waste treatment, and agriculture. However, due to the lack of accurate measurement techniques the exact diversity of prokaryotes is unknown and unknowable at any scale in any environment and remains highly speculative.

To fill this gap, today's microbiologists are seeking a similar grail as that of eighteenth-century biologists studying plant and animal species: a method for counting the number of species. However, the quest is much more challenging for the microbiologists: microbes are tiny and often cannot be distinguished morphologically, the number of species is huge, and species definition remains problematic (Gevers et al.,).

A first way to depict microbial diversity is measuring microbial richness, in a sample, i.e. the number of species in said sample. However, attempts at species estimation suffers from a lack of information about rare species, as the contribution of rare species is overwhelmed by that of common ones (Pedros-Alio et al.,).

To improve microbial diversity estimation, the lack of information about rare species, has been compensated by assumptions about the bacterial species abundance distribution (SAD). However, These assumptions are crude extrapolations from what is known about eukaryotic organisms and remain unverifiable in the microbial world.

For these reasons, the construction of an accurate microbial richness estimator is problematic (Curtis et al., Dunbar et al., Gans et al., Hong et al., Loisel et al., Torsvik et al., Venter et al.,). For example, estimates of the number of microbial species vary between $10^4$ and $10^7$ in 1 g of pristine soil, based on the same data but different assumptions for the SAD (Gans et al., Torsvik et al.,).

As a consequence, measuring richness is simply not rich enough to estimate biological diversity. However, other diversity quantities exist that give more weight to common than to rare species, like the Shannon diversity index (Shannon) and the Simpson diversity index (Simpson).

To estimate the extent of microbial diversity, new genetic techniques have revealed extensive microbial diversity that has previously gone undetected. These techniques are based on nucleic acids, allow detection of the vast majority of microbes and give an accurate glimpse of the biodiversity in the environment (Pace et al.,).

For example these new techniques consist of targeting the 16S ribosomal RNA gene, and carrying out Denaturing Gradient Gel Electrophoresis (DGGE), Terminal Restriction Fragment Length Polymorphism (T-RFLP), Ribosomal Intergenic Spacer Analysis (RISA) and Single Strand Conformation Polymorphism (SSCP).

These techniques provide fingerprinting profiles, which can be considered as an image of a microbial ecosystem. Such a picture of the bacterial community is given by discrete bands or peaks which emerge from a subpeak background, wherein peaks are associated to numerically dominant sequences and subpeak background are considered as coming from a large number of rare sequences, generally omitted from the analysis.

A bibliographic survey showed that one-third of microbial fingerprinting publications attempt to estimate the species richness. From a literature survey on September 7, 2006, 1466 research articles have been identified in the ISI Web of Science since 1993 that used fingerprints of microbial communities. A random subset of 200 papers was analyzed in full text. Twenty-three percent of the papers interpreted the number of bands as a measure of species richness, and 10% of the papers applied the Simpson or the Shannon index to fingerprinting data.

Richness estimation is typically based on the number of peaks in the fingerprinting profile. However, a simulation study of the DGGE and SSCP techniques demonstrated that the number of peaks is a poor measure of richness (Loisel et al.,). In fact, when richness increases, the number of peaks saturates, the sum of the peak areas decreases, and the background area increases.

Based on this observation, Loisel et al. have developed a new tool for estimating diversity on the basis of fingerprinting profiles obtained from SSCP technique. The authors make use of a method described by Curtis et al. This method estimates bacterial diversity of prokaryotic communities by calculating the ratio between two variables : the total number of individuals in a sample and the abundance of the most abundant species in said sample.

Nevertheless, as recognised by the authors, two hypotheses limit the use of this method to estimate bacterial diversity:

(i) the distribution of biological species abundances must follow a lognormal law, and
(ii) the less abundant species must comprise only one individual.

However, the distribution of species abundances in a sample to analyse is unknown, and does not meet necessarily the two criteria above.

Then, there exists a need for new methods for measuring biological diversity in a sample which can apply to any sample, and for example to a sample wherein the distribution of biological species is not lognormal, and/or to a sample wherein the less abundant species does not comprise only one individual.

The present invention fulfils these needs and further provides other related advantages.

## Summary of the invention

The invention is firstly directed to a computer-implemented method for measuring biological diversity in a sample. The method comprises steps essentially involving two components, (a) a migration of a sample comprising molecules on a surface area, whereby molecules migrate on a surface area according to their nucleotide or aminoacid sequence and (b) method steps for the diversity measurement, which are preferably performed through the working of one or several computer program(s).

The said method comprises the following successive steps:

a) submitting a sample to migration on the surface area of a migration substrate, wherein the said sample comprises one or more molecules selected from the group consisting of nucleic acids and proteins, wherein the said molecules belong to one or more species, and wherein molecules which belong to the same species have an identical nucleotide or aminoacid sequence;

b) generating a fingerprinting profile from the migration pattern of the molecules on the surface area of the said migration substrate, the said fingerprinting profile comprising a number (n) of peaks ($P_1, P_2, ..., P_n$) and a background (B);

c) from the fingerprinting profile generated at step b), determining respectively :

(i) the surface area ($a'_1, a'_2, ..., a'_n$) for each peak ($P_1, P_2, ..., P_n$) contained in the said fingerprinting profile;
(ii) the surface area (b') of the background (B);

d) normalising the values ($a'_1, a'_2, ..., a'_n$) and b' into the values ($a_1, a_2, ..., a_n$) and b, using the formulas:

$$a_p = \frac{a'_p}{\sum_{p=1}^{n} a'_p + b'} \quad \text{and} \quad b = \frac{b'}{\sum_{p=1}^{n} a'_p + b'}$$

such that

$$\sum_{p=1}^{n} a_p + b = 1;$$

e) estimating the logarithmic Simpson diversity index (Z) of the said sample by calculating the estimated logarithmic Simpson diversity index ($Z_1$) by the formula:

$$Z_1 = -\ln \sum_{p=1}^{n} a_p^2 .$$

It has been found according to the invention that the experimental measure of diversity of a sample, can be mathematically corrected through a correction factor (C) determined by applying the method of the invention to a set of computer-generated samples, whom diversity is known.

In a preferred embodiment of the method according to the invention the value $Z_1$ is corrected by a correcting factor (c).

Accordingly, the invention also concerns a computer-implemented method for measuring biological diversity in a sample, comprising the following steps :

A) providing a set of computer-generated samples, each of said computer-generated samples comprising a known number of species (S), and each of said species having a known relative abundance. The relative abundance of species (1, 2,..., S) is denoted by $(n_1, n_2,..., n_S)$,

B) determining for each computer-generated sample the logarithmic Simpson diversity index Z, using the formula:

$$Z \square \square \ln \sum_{i \square 1}^{S} n_i^2 \ ,$$

C) determining for each computer-generated sample the value $Z_1$, by first generating a corresponding fingerprinting profile, and next applying step c) to step e) as defined above,

D) calculating the correction factor (c) by performing a linear regression between the set of pairs $(Z; Z_1)$ obtained in steps B) and C). The correction factor c satisfies:

$Z \square cZ_1$,

E) providing a biological sample, comprising one or more molecules selected from nucleic acids and proteins, said molecules belonging to one or more species, and wherein molecules belonging to a same species have identical nucleotide or aminoacid sequence,

F) determining the value $Z_1$ of said sample according to step b) to step e) as defined above,

G) calculating the value $Z_{1c}$ so as

$Z_{1c} \square cZ_1$,

wherein the value $Z_{1c}$ is the corrected estimated logarithmic Simpson diversity index and therefore a measure of the biological diversity of said sample.

## Description ot drawings

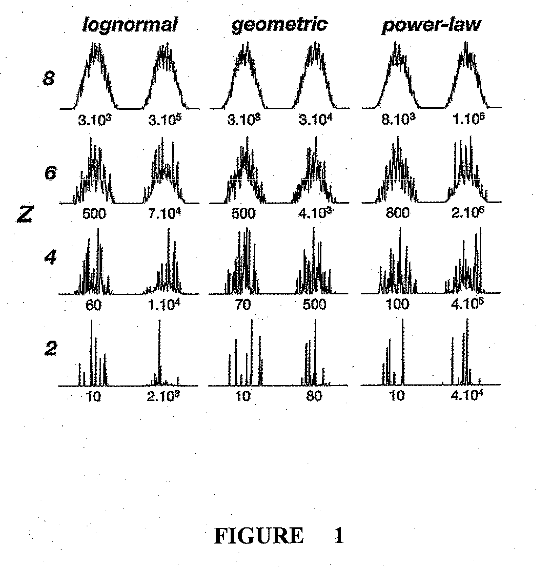

Figure 1. Simulated SSCP fingerprinting profiles with logarithmic Simpson diversity index Z ranging from 2 to 8. The profiles were generated using lognormal, geometric, and power-law SADs. The number of simulated species is indicated under each profile. For a constant logarithmic Simpson diversity index, the fingerprinting profiles look similar, while the species richness varies widely.

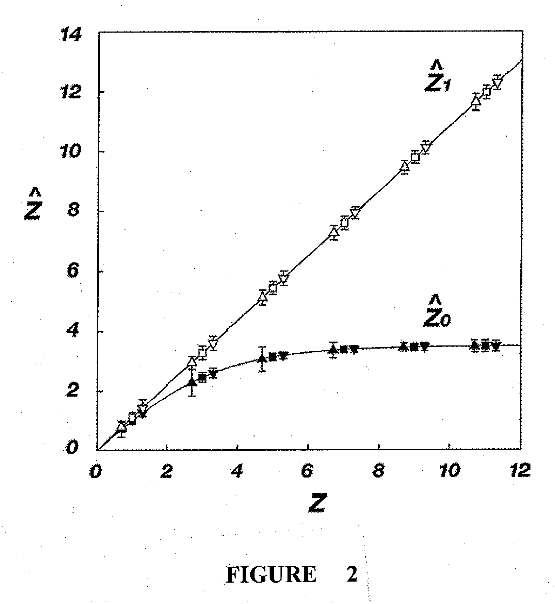

Figure 2. Accuracy of the logarithmic Simpson diversity estimator $Z_1$ for simulated fingerprints compared to the estimator $Z_0$ that neglects the background. The error bars were calculated in the ranges 0-2, 2-4, 4-6, 6-8, 8-10, and 10-12 for a total of 5000 communities for each of the three SADs (▲ lognormal, ■ geometric, ▼ power law). $Z_1$ overestimates the diversity by 8%. This bias can be easily corrected, as was done for all experimental results (Table 1). $Z_0$ saturates for diversified communities.

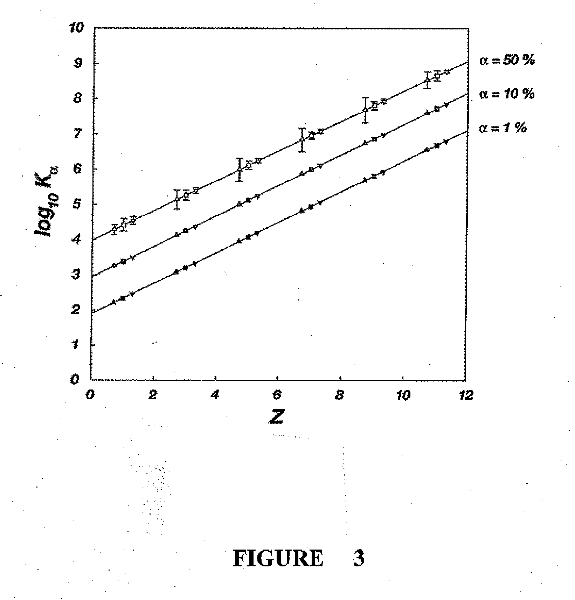

Figure 3. Correlation between the logarithmic Simpson diversity index Z and $K_\alpha$, the number of sequences required to obtain a fraction $\alpha$ of redundant DNA in a metagenomic survey. For each of the three SADs (▲ lognormal, ■ geometric, ▼ power law) 5000 communities were generated, and a linear fit log10 $K_\alpha$ vs. Z was computed for $\alpha$ = 1% (plain symbols), $\alpha$ = 10% (halftone) and $\alpha$ = 50% (open). The error with respect to this fit is very small for small $\alpha$ (0.01 for $\alpha$ = 1%, and 0.1 for $\alpha$ = 10%). For $\alpha$ = 50% error bars are shown. 12

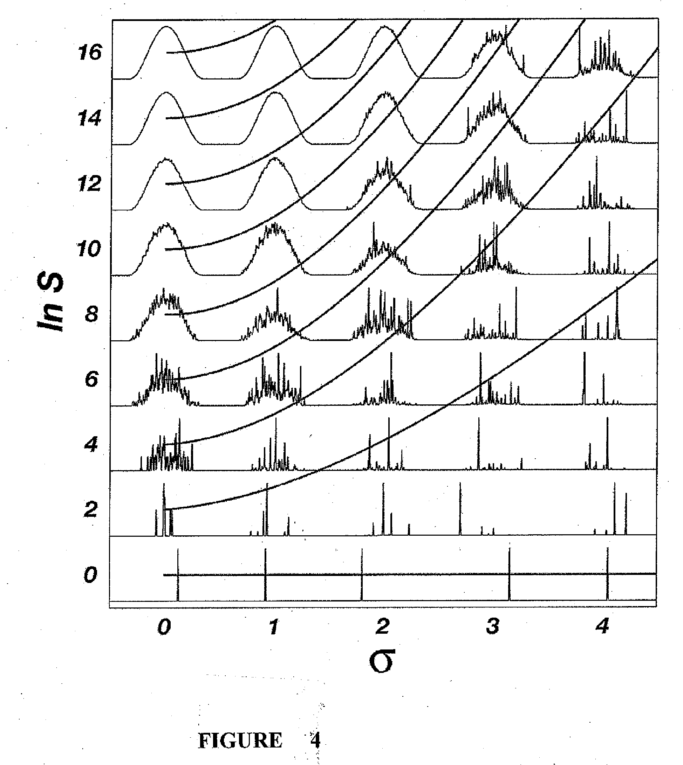

Figure 4: Simulated SSCP profiles using a lognormal SAD. The profile generated with S species and lognormal parameter σ is centred at coordinates (σ; In S). It has to be noticed that for given S and σ the SSCP profile is a random object, and this figure therefore only shows one possible realization. In particular for large σ, the profile changes from realization to realization. The curves are the isolines of the expected value of the logarithmic Simpson diversity index Z. For a given isoline, the corresponding index Z can be read off on the In S axis, as for a lognormal community with σ = 0, $n_i$ = 1/S for all species i, and Z = In S.

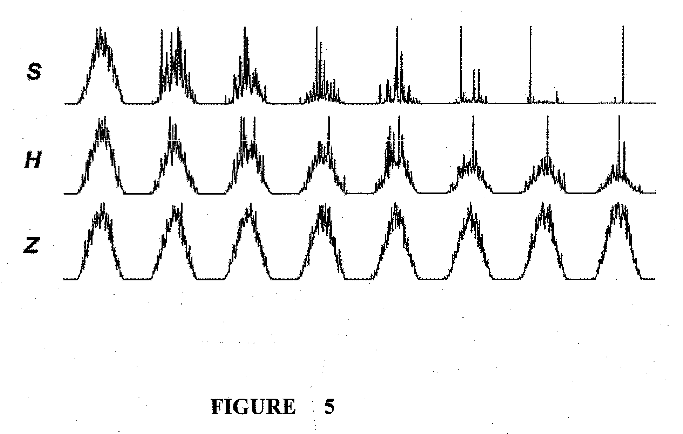

Figure 5: Simulated SSCP profiles with constant diversity. The first row corresponds to constant number of species S (S = 2981 or In S = 8.00), the second row to constant Shannon diversity index H (H = 8.00 $\pm$ 0.01 ) and the third row to constant logarithmic Simpson diversity index Z (Z = 8.00 ± 0.01). The parameter σ increases from left to right, ranging from 0 to 4.

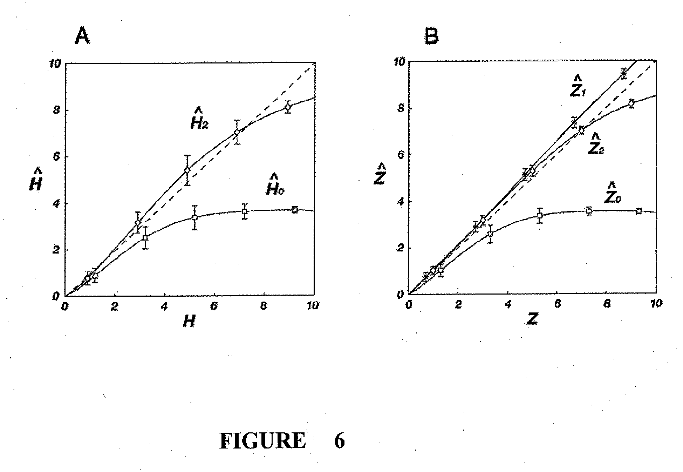

**Figure 6:** Accuracy of the diversity index estimators. For each of the three SADs (log-normal, geometric and power-law), 5000 communities were generated randomly together with their SSCP profile, and the different estimators were evaluated. For the estimator $Z_1$, for example, a polynomial of degree five was fitted to the data (Z; $Z_1$), and the error with respect to this fit was calculated in the ranges 0-2, 2-4, 4-6, 6-8 and 8-10. (A) The Shannon diversity index H and its estimators $H_0$ and $H_2$. (B) The Simpson diversity index Z and its estimators $Z_0$, $Z_1$ and $Z_2$.

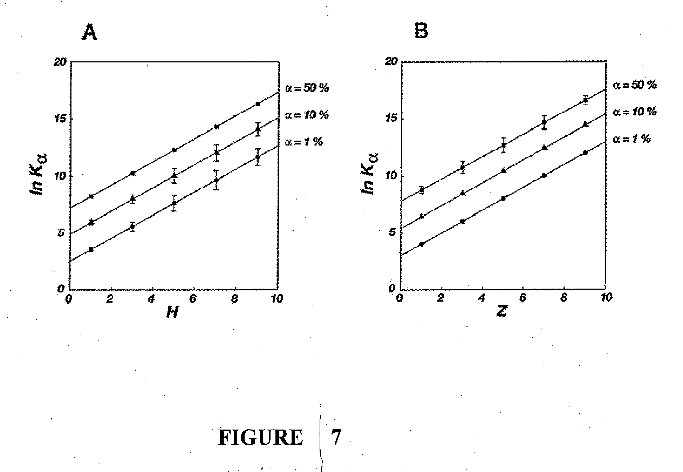

**Figure 7:** Diversity indices and sequencing effort. For each of the three SADs (lognormal, geometric and power-law), 5000 communities were generated randomly together with their rarefaction curve. (A) The sequencing effort ln $K_\alpha$ vs. the Shannon diversity index H, for $\alpha = 0.01$, $\alpha = 0.1$ and $\alpha = 0.5$. (B) The sequencing effort ln $K_\alpha$ vs. the logarithmic Simpson diversity index Z for $\alpha = 0.01$, $\alpha = 0.1$ and $\alpha = 0.5$.

## Detailed description of the invention

The inventors have carried out a new method for measuring diversity in a sample, which can apply to any sample, and in particular to a sample whom species distribution is unknown.

As mentioned above, fingerprinting profiles are widely used for providing an image of a microbial ecosystem. The inventors have investigated how such fingerprinting profiles harbour diversity information, and this investigation has allowed the development a new method for measuring diversity in a sample.

The inventors have generated simulations of fingerprints, focusing on the quantitative analysis of SSCP fingerprints (see examples below). The various experimental profiles have been faithfully simulated by assuming that every species contributes a sharp peak with an area proportional to its abundance, thus neglecting biases due to DNA extraction, PCR amplification, and multiple copies of the targeted gene. Because the inventors have full access to the simulation inputs, they can investigate what information about the microbial community is encoded in the profiles.

To this end, the inventors have generated profiles from several communities obeying to a large variety of SADs, including lognormal, geometric, and power-law distributions.

Considering a community of S species, and denoting by $(n_1, n_2,..., n_S)$ the relative abundance of species (1, 2, ..., S), the inventors used the logarithmic Simpson diversity index Z defined by the following formula:

$$Z = -\ln \sum_{i=1}^{S} n_i^2 .$$

The logarithmic Simpson diversity index Z is closely related to the Simpson concentration index C, as shown in the formula hereunder :

$$Z = -\ln C \quad \text{with} \quad C = \sum_{i=1}^{S} n_i^2 ,$$

and is therefore equivalent to common diversity measures such as 1-C and 1/C.

On the basis of the data above, the inventors have shown that the logarithmic Simpson diversity index *Z* is encoded in the fingerprinting profiles independently of the number of species and their abundance distributions.

Indeed, the inventors have shown that profiles corresponding to communities with constant Z appear very similar over a large range of number of species and SADs, especially for highly diverse assemblages. The variation in richness of communities yielding similar snapshots can be as large as a factor of 1000. This property is less satisfied for a constant Shannon diversity index and completely lost for a constant richness. The tight association between Simpson diversity and fingerprints is illustrated in Figure 1.

Based on the above results, the inventors have built an explicit estimator of the Simpson diversity index using experimental fingerprints.

The authors have considered fingerprinting profile with total area under the profile equal to 1. The number of peaks have been denoted by n, and the relative area of peaks $(P_1, P_2, ..., P_n)$ by $(a_1, a_2, ..., a_n)$. The authors then estimated the logarithmic Simpson diversity index Z by the following formula:

$$Z_1 \square \square \ln \sum_{p \square 1}^{n} a_p^2 .$$

The inventors have shown that the accuracy of this estimator for profiles of simulated communities is very good, with a mean square error of 0.2 regardless of the complexity of the community (Figure 2).

In addition, the inventors have shown that this estimator is independent of the species abundance distribution (SAD). This latter property renders the estimator very useful, as SAD assumptions, that break down richness estimators previously available (Curtis et al., Dunbar et al., Gans et al., Hong et al., Loisel et al., Torsvik et al., Venter et al.,), are not needed with the estimator built by the inventors.

As a consequence, using this new estimator the inventors have carried out a new method for measuring diversity in a sample, which can apply to any sample, and in particular to a sample whom species distribution is unknown.

Accordingly, an object of the present invention consists of a computer-implemented method for measuring biological diversity in a sample comprising the following successive steps:

a) submitting a sample to migration on the surface area of a migration substrate, wherein the said sample comprises one or more molecules selected from the group consisting of nucleic acids and proteins, wherein the said molecules belong to one or more species, and wherein molecules which belong to the same species have an identical nucleotide or aminoacid sequence;

b) generating a fingerprinting profile from the migration pattern of the molecules on the surface area of the said migration substrate, the said fingerprinting profile comprising a number (n) of peaks ($P_1, P_2, ..., P_n$) and a background (B);

c) from the fingerprinting profile generated at step b), determining respectively :

(i) the surface area ($a'_1, a'_2, ..., a'_n$) for each peak ($P_1, P_2, ..., P_n$) contained in the said fingerprinting profile;
(ii) the surface area (b') of the background (B);

d) normalising the values ($a'_1, a'_2, ..., a'_n$) and b' into the values ($a_1, a_2, ..., a_n$) and b, using the formulas:

$$a_p \square \frac{a'_p}{\sum_{p \square 1}^{n} a'_p \square b'} \quad \text{and} \quad b \square \frac{b'}{\sum_{p \square 1}^{n} a'_p \square b'}$$

such that

$$\sum_{p \square 1}^{n} a_p \square b \square 1 .$$

e) estimating the logarithmic Simpson diversity index (Z) of the said sample by calculating the estimated logarithmic Simpson diversity index ($Z_1$) by the formula:

$$Z_1 \square \square \ln \sum_{p \square 1}^{n} a_p^2 .$$

Generally, as it is known in the art, the Simpson diversity index reflects the probability of drawing individuals belonging to the same species when randomly drawing two individuals from the community.

Generally, at step e), the one skilled in the art may use other formulas as a measure of the biological diversity.

According to the computer-implemented method above, the one skilled in the art may thus perform step e) using other embodiments known in the art for calculating the biological diversity based on the values ($n_1, n_2, ..., n_S$) for the relative species abundances, provided that it is made use of the values ($a_1, a_2, ..., a_n$) for the relative peak areas.

In certain embodiments, the biological diversity is defined using the formula:

$$D^{(1)} = \sum_{i=1}^{S} n_i^x$$

The corresponding biological diversity estimator is calculated at step e) using the following formula (1) :

$$D_1^{(1)} = \sum_{p=1}^{n} a_p^x \,,$$

wherein :

- $n_i$ consists of the value of relative abundance of species i,
- $a_p$ consists of the value of relative area of peak p, and
- x consists of a real number comprised between 1.5 and 3.

In other embodiments, the biological diversity is defined using the formula:

$$D^{(2)} = 1 - \sum_{i=1}^{S} n_i^x$$

The corresponding biological diversity estimator is calculated at step e) using the following formula (2) :

$$D_1^{(2)} = 1 - \sum_{p=1}^{n} a_p^x \,,$$

wherein :

- ni consists of the value of relative abundance of species i,
- $a_p$ consists of the value of area of peak p, and
- x consists of a real number comprised between 1.5 and 3.

In further embodiments, the biological diversity is defined using the formula:

$$D^{(3)} = \frac{1}{1 - \sum_{i=1}^{S} n_i^x}$$

The corresponding biological diversity estimator is calculated at step e) using the following formula (3) :

$$D_1^{(3)} = \frac{1}{1 - \sum_{p=1}^{n} a_p^x} \,,$$

wherein :

- $n_i$ consists of the value of relative abundance of species i,
- $a_p$ consists of the value of area of peak p, and

- x consists of a real number comprised between 1.5 and 3.

In still further embodiments, the biological index is defined using the formula:

$$D^{(4)} = \ln \sum_{i=1}^{S} n_i^x$$

The corresponding biological diversity estimator is calculated at step e) using the following formula (4) :

$$D_1^{(4)} = \ln \sum_{p=1}^{n} a_p^x ,$$

wherein:

- In denotes the Napierian logarithm (also termed "natural logarithm")
- $n_i$ consists of the value of relative abundance of species i,
- $a_p$ consists of the value of area of peak p, and
- x consists of a real number comprised between 1.5 and 3.

According to any one of the formulae (1) to (4) above, x has a value of preferably at least 1.8.

According to any one of the formulae (1) to (4) above, x has a value of preferably at most 2.2.

According to any one of the formulae (1) to (4) above, x is preferably equal to 2.

According to a preferred embodiment of step a) of the computer-implemented method above, the said step a) comprises the following steps :

a1) providing a sample comprising one or more molecules selected from nucleic acids and proteins, said molecules belonging to one or more species, and wherein molecules belonging to a same species have identical nucleotide or aminoacid sequence ,

a2) submitting said sample to a separation step on the surface area of a separation substrate, whereby molecules migrate on the surface area, along a X-axis at different locations according to their nucleotide or amino acid sequence,

a3) dividing the surface area in a series of blocks, along the said X-axis, each block having the same surface, and

a4) assigning a location value for each block contained in the surface area, wherein the location value consists of the location of each block, along the said X-axis, respective to a unique predetermined point of origin in the surface area, so as the blocks are ordered along the X axis,

According to a preferred embodiment of step b) of the computer-implemented method above, step b) comprises the following steps :

b1) quantifying the number of molecules at each block, to obtain an intensity value, wherein the intensity value is representative of the number of molecules in each block,

b2) generating a number of experimental ordered pairs equal to the number of blocks, each ordered pair having an X-coordinate and an Y-coordinate, and wherein :

(i) the location value of one block constitutes the X-coordinate of one ordered pair, and

(ii) the intensity value of the signal corresponding to the block cited in (i), constitutes the Y-coordinate of the ordered pair cited in (i),

b3) generating a two dimensional surface area defined by an X-axis and an Y-axis,

b4) representing each ordered pair as a point onto the two-dimensional surface area, using the X-coordinate and the Y-coordinate of each ordered pair for each point, respective to the X-axis and Y-axis, and

b5) connecting by a line each point to the one having the closest X-coordinate, to obtain a curve comprising a number (n) of peaks, and wherein :

(i) each peak is separated from another peak by a valley,

(ii) each peak comprises a top,

(iii) each valley comprises a bottom,

According to a preferred embodiment of step c) of the computer-implemented method above, the said step c) comprises the following steps :

c1) determining the X-coordinate of each bottom on the two-dimensional surface area,
c2) connecting by a line each bottom to the one having the closest X-coordinate, to obtain a baseline,
c3) determining for each of the (n) peaks the surface area delimited by the contours of the peak and the baseline,
c4) assigning a number (n) of values ($a'_1$, $a'_2$, ... $a'_n$), value $a'_p$ being representative of the area of peak p,
c5) determining the surface area between the baseline and the X-axis from the lower limit to the upper limit, said surface consisting of a background, and
c6) assigning a value (b') representative of the surface area between the base line and the X-axis,

As already described earlier in the present specification; step d) of the computer-implemented method according to the invention consists of normalising the values ($a'_1$, $a'_2$, ..., $a'_n$) and b' into the values ($a_1$, $a_2$, ..., $a_n$) and b, using the formulas:

$$a_p = \frac{a'_p}{\sum_{p=1}^{n} a'_p + b'} \quad \text{and} \quad b = \frac{b'}{\sum_{p=1}^{n} a'_p + b'}$$

such that

$$\sum_{p=1}^{n} a_p + b = 1.$$

Then, at step e), the biological diversity value of the sample is determined, namely the logarithmic Simpson diversity index (Z) is estimated by calculating the estimated logarithmic Simpson diversity index ($Z_1$) and by using the normalised values obtained at step d) above.

A first technical advantage of the diversity measurement method according to the invention lies in the fact that its implementation does not require the knowledge of the distribution of the abundance of the species contained in the sample that is studied, and then, can be applied to any sample, in contrast to a prior state of the art method of analysis (Curtis et al.,) which needs that:

- (i) the distribution of biological species follows a lognormal law, and
- (ii) the less abundant species comprises only one individual.

A further technical advantage of the method of the invention is its high sensitivity. Indeed, the accuracy of this estimator for profiles of computer-generated communities is very good, especially when determining the biological diversity value according to formula (4) above and with x = 2, i.e. the logarithmic Simpson diversity index Z. The mean square error is 0.2 regardless of the complexity of the community (figure 2). The estimation is robust with respect to added noise in the computer-generated SSCP profiles and for less precise computations of the peak areas. The present method is more accurate than the method of the state of the art using the Shannon index (Figure 6).

Without willing to be bound by any particular theory, the inventors believe that this difference might be related to the observation that fingerprinting patterns that can be easily distinguished by the present method, can have the same Shannon diversity index (Figure 5). Shannon and Simpson diversity have been often estimated from fingerprints, but the present method, is fundamentally different because it takes into account the background area under the peaks.

Indeed, common practice (Fromin et al.,) eliminates the background by normalising the values ($a'_1$, $a'_2$, ..., $a'_n$) into the values ($a''_1$, $a''_2$, ..., $a''_n$), using the formula:

$$a''_p \, \square \, \frac{a'_p}{\sum\limits_{p \square 1}^{n} a'_p} \, ,$$

such that:

$$\sum\limits_{p \square 1}^{n} a''_p \, \square \, 1,$$

leading to the estimator:

$$Z_0 \, \square \, \square \ln \sum\limits_{p \square 1}^{n} a''^2_p \, .$$

It can be proven that this estimator is bounded from above by ln P, saturating therefore at $Z_0 \approx 3$. In contrast, the estimator $Z_1$ is almost perfectly linear up to Z= 12 (Figure 2).

Thus, the present method enables an easy estimation of microbial diversity. The proposed measurement is rapid and accurate. The method according to the invention has multiple applications as shown in Example 1. For example, it can be used for qualifying biotopes, and investigating environmental changes. This method can also guide community selection in metagenomic approaches as shown in example 4.

A further technical advantage of the method of the invention consists of the possibility to perform automatically steps (a) to (e) of the method, or in some embodiments (a3) to (e) of the method, for example by designing a computer program which performs automatically steps (a) to (e), or in some embodiments (a3) to (e), and then displays the results of estimation of biological diversity of the sample that is under analysis.

As it will be explained in detail further in the present specification, the identification method of the invention can be used for estimating biological diversity, and for example microbial diversity in any sample such as soils, compost and gut; anaerobic digester; feces, waste water, and sea water; and root, milk, or drinking water.

According to the latter preferred embodiment, the method of the invention comprises steps essentially involving two main general features, respectively, (1) a migration of a sample comprising molecules on a surface area, whereby molecules migrate on a surface area according to their nucleotide or aminoacid sequences and (2) method steps for the diversity estimation, which are preferably performed through the working of one or several computer program(s).

In preferred embodiments of the computer-implemented method of the invention, this or these computer program (s) perform(s) five functions:

(1) The said computer program(s) contain instructions for generating a digital image of the migration pattern of the one or more molecules contained in the sample under analysis, whereby a dataset corresponding to the said migration pattern is generated and then stored in a computer storage mean, e.g. a computer memory or a computer disk. Generally, each data of the said dataset consists of the combination of at least two pertinent numerical values of the smallest analysed surface area of the migration substrate, wherein (i) the first pertinent numerical value consisting of the location value of the said smallest analysed surface area along the axis of molecules migration on the said migration substrate and (ii) the second numerical value consists of the magnitude of the signal generated by the migrated molecules, e.g. the intensity of a fluorescence signal that is generated by the migrated molecules that are located at the said smallest analysed surface area. Thus, the location value consists of a location value along the axis of migration, which may also be termed X-axis herein, of the sample molecules, on the surface area of the migration substrate. In some embodiments, the smallest analysed surface area of the said migration substrate, for generating the said dataset, consists of a pixel contained in the digitised migration substrate, or alternatively a set of pixels defining the said smallest analysed surface area of the said migration substrate.

(2) Then, the said computer program(s) contain instructions for generating a graph comprising an X-axis and an Y-axis, the Y-axis being representative of the magnitude of a signal by the migrated sample molecules (e.g. the intensity value of a signal), corresponding to the presence of molecules from said sample on the migration surface area, and the X-axis being representative of the distance from a point of origin on the surface area of the migration substrate, the said graph comprising a curve comprising peaks and valley, and wherein each peak corresponds to a maximum intensity of the signal for a location along the migration axis (e.g. X-axis),

(3) Then, the said computer program(s) contain instructions for separating the peaks from a background,

(4) Then, the said computer program(s) contain instructions for assigning values ($a'_1$, $a'_2$, ..., $a'_n$) representative of the surface area of the peaks and a value ($b'$) representative of the surface area of the background,

(5) Then, the said computer program(s) contain instructions for normalising the values ($a'_1$, $a'_2$, ..., $a'_n$) and $b'$ into a values ($a_1$, $a_2$, ..., $a_n$), so that:

$$\sum_{p=1}^{n} a_p + b = 1,$$

and

(6) Then, the said computer program(s) contain instructions for calculating the estimated logarithmic Simpson diversity index ($Z_1$) using the following relationship:

$$Z_1 = -\ln \sum_{p=1}^{n} a_p^2 .$$

**Definitions according to the invention**

The "sample" as intended herein, is a biological sample comprising one or more molecules selected from nucleic acids and proteins. The term "nucleic acid" designates DNA and RNA molecules, and the term "protein" also encompasses peptides and polypeptides. A "sample" can be derived from any biotope. Non limiting examples of biotopes are for example soil, compost and gut; anaerobic digester; feces, waste water, and sea water; root, milk, or drinking water.

The method according to the invention makes use of nucleic acids and proteins to estimate biological diversity in a sample and, in a sample according to the invention, molecules having identical nucleotide or aminoacid sequence are considered as belonging to a same species. Accordingly, nucleic acids or proteins able to discriminate microbial species are preferably used in the method according to the invention. For example, the bacterial 16S rRNA gene, the bacterial housekeeping genes rpoB, recA, the fungal 18S rRNA gene, etc. can be used.

By "measuring biological diversity" it is intended herein a measure which is representative of the biological diversity of a sample, or in other words an estimation of the biological diversity in a sample. In the specification, the terms "measure of biological diversity" and "estimation of biological diversity" are alternatively used to designate this measure.

By "migrating properties", it is intended herein the ability of a set of molecules consisting of aminoacids or nucleic acids, which are contained in the sample, to shift along the surface area of a substrate, from an initial location wherein it is deposited on the said substrate, to another location of the surface area of the said substrate, the shift of each molecule of the said sample being caused by the physical or chemical properties of each of the molecule, such as its electric charge, its molecular mass, its acid/basic properties, etc. The expression "migrating properties" also encompasses the behaviour of a given nucleic acid on any separation substrate, such as a capillary electrophoresis system or any other separation substrate. Indeed, when the "migration properties" of a given nucleic acid are determined in the method of the invention, on capillary electrophoresis system, the said migrating properties may be expressed as the retention time of the said given nucleic acid in the said capillary electrophoresis system.

Similarly, the "migration properties" of a given peptide encompasses the behaviour of a given peptide on any separation substrate, such as an HPLC chromatography column or any other separation substrate. Indeed, when the "migration properties" of a given peptide are determined, in the method of the invention, on an HPLC separation substrate, the said migrating properties may be expressed as the retention time of the said given peptide on the said HPLC separation substrate.

As used herein, a "separation substrate" or a "migration substrate" refers to a substrate having a surface area that is used for performing the separation step which is referred to above. According to the invention, a separation substrate encompasses chromatography substrates, including Thin Layer Chromatography (TLC) substrates, suitable substrates for iso-electrofocusing separation, or any other substrate which allows the separation of peptides or proteins according to their physical and/or chemical properties, such as, for example, any chromatography substrate, including HPLC ("High Performance Liquid Chromatography"), FPLC (Fast Protein Liquid Chromatography"), gel filtration chromatography, ion exchange chromatography, reverse phase chromatography or affinity chromatography. A separation substrate also encompasses electrophoresis substrate such as capillary electrophoresis substrate or any other substrate which allows the separation of nucleic acids according to their physical and/or chemical properties.

According to step b) of the method above, or in some embodiments step b5) of the method above, a curve comprising a number of peaks and valleys, is obtained on a graph.

The terms peaks and valleys can be defined one by reference to the other, as follows.

A "peak" is constituted by the part of the curve located between two adjacent bottoms of valleys and a "valley" is constituted by the part of the curve located between two adjacent tops of peaks.

The top of a peak is the point of the curve in the peak presenting the greater ordinate value, and the bottom of a valley is the point of the curve of a valley presenting the lower ordinate value.

At step a) of the method above, or in some embodiments at step a2) of the method above, a sample is submitted to a separation step on the surface area of a separation substrate.

An essential technical advantage of the method according to the invention is that the method used to separate the molecules contained in the sample, at step a) of the method, or in some embodiments at step a2) of the method, is not limiting. A wide variety of techniques for separating nucleic acids and proteins well known to those skilled in the art can be used. Preferred separation techniques that can be used to perform step a), or in some embodiments step a2), are described hereunder.

## Preferred separation techniques used at step a), or at step a2)

### - High- Performance Liquid Chromatography (HPLC)

High-Performance liquid chromatography (HPLC) is a chromatographic separations technique to separate compounds that are dissolved in solution. HPLC instruments consist of a reservoir of mobile phase, a pump, an injector, a separation column, and a detector. Compounds are separated by injecting an aliquot of the sample mixture onto the column. The different components in the mixture pass through the column at different rates due to differences in their partitioning behaviour between the mobile liquid phase and the stationary phase.

Recently, IP-RO-HPLC on non-porous PS/DVB particles with chemically bonded alkyl chains have been shown to be rapid alternatives to capillary electrophoresis in the analysis of both single and double-strand nucleic acids providing similar degrees of resolution (Huber et al, Anal. Biochem. 212:351, 1993; Huber et al., 1993, Nuc. Acids Res. 21:1061; Huber et al., Biotechniques 16:898, 1993). In contrast to ionexchange chromatography, which does not always retain double-strand DNA as a function of strand length (Since AT base pairs interact with the positively charged stationary phase, more strongly than GC base-pairs), IP-RP-HPLC enables a strictly size-dependent separation.

A method has been developed using 100 mM triethylammonium acetate as ion-pairing reagent, phosphodiester oligonucleotides could be successfully separated on alkylated non-porous 2.3 $\mu$M poly(styrenedivinylbenzene) particles by means of high performance liquid chromatography (Oefner et al., Anal. Biochem. 223:39, 1994). The technique described allowed the separation of PCR products differing only 4 to 8 base pairs in length within a size range of 50 to 200 nucleotides.

### Electrophoresis

Electrophoresis is a separations technique that is based on the mobility of ions (or DNA as is the case described herein) in an electric field. Negatively charged DNA charged migrate towards a positive electrode and positively-charged ions migrate toward a negative electrode. For safety reasons one electrode is usually at ground and the other is biased positively or negatively. Charged species have different migration rates depending on their total charge, size, and shape, and can therefore be separated. An electrode apparatus consists of a high-voltage power supply, electrodes, buffer, and a support for the buffer such as a polyacrylamide gel, or a capillary tube. Open capillary tubes are used for many types of samples and the other gel supports are usually used for biological samples such as protein mixtures or DNA fragments.

### Capillary Electrophoresis (CE)

Capillary electrophoresis (CE) in its various manifestations (free solution, isotachophoresis, isoelectric focusing, polyacrylamide gel, micellar electrokinetic "chromatography") is developing as a method for rapid high resolution separations of very small sample volumes of complex mixtures. In combination with the inherent sensitivity and selectivity of MS, CE-MS is a potential powerful technique for bioanalysis. In the present invention, the interfacing of these two methods will lead to superior DNA sequencing methods that eclipse the current rate methods of sequencing by several orders of magnitude.

The correspondence between CE and electrospray ionization (ESI) flow rates and the fact that both are facilitated by (and primarily used for) ionic species in solution provide the basis for an extremely attractive combination. The combination of both capillary zone electrophoresis (CZE) and capillary isotachophoresis with quadrapole mass spectrometers based upon ESI have been described (Olivares et al., Anal. Chem. 59:1230, 1987; Smith et al., Anal. Chem. 60:436, 1988; Loo et al., Anal. Chem. 179:404, 1989; Edmonds et al., J. Chroma. 474:21, 1989; Loo et al., J. Microcolumn Sep. 1:223, 1989; Lee et al., J. Chromatog. 458:313, 1988; Smith et al., J. Chromatog. 480:211, 1989; Grese et al., J. Am. Chem. Soc. 111:2835, 1989). Small peptides are easily amenable to CZE analysis with good (femtomole) sensitivity.

The most powerful separation method for DNA fragments is polyacrylamide gel electrophoresis (PAGE), generally in a slab gel format. However, the major limitation of the current technology is the relatively long time required to perform the gel electrophoresis of DNA fragments produced in the sequencing reactions. An increase magnitude (10-fold) can be achieved with the use of capillary electrophoresis which utilize ultrathin gels. In free solution to a first approximation all DNA migrate with the same mobility as the addition of a base results in the compensation of mass and charge. In polyacrylamide gels, DNA fragments sieve and migrate as a function of length and this approach has now been applied to CE. Remarkable plate number per meter has now been achieved with cross-linked polyacrylamide ($10^7$ plates per meter, Cohen et al., Proc. Natl. Acad. Sci., USA 85:9660, 1988). Such CE columns as described can be employed for DNA sequencing. The method of CE is in principle 25 times faster than slab gel electrophoresis in a standard sequencer. For example, about 300 bases can be read per hour. The separation speed is limited in slab gel electrophoresis by the magnitude of the electric field which can be applied to the gel without excessive heat production. Therefore, the greater speed of CE is achieved through the use of higher field strengths (300 V/cm in CE versus 10 V/cm in slab gel electrophoresis). The capillary format reduces the amperage and thus power and the resultant heat generation.

Smith and others (Smith et al., Nuc. Acids. Res. 18:4417, 1990) have suggested employing multiple capillaries in parallel to increase throughput. Likewise, Mathies and Huang (Mathies and Huang, Nature 359:167, 1992) have introduced capillary electrophoresis in which separations are performed on a parallel array of capillaries and demonstrated high through-put sequencing (Huang et al., Anal. Chem. 64:967, 1992, Huang et al., Anal. Chem. 64:2149, 1992). The major disadvantage of capillary electrophoresis is the limited amount of sample that can be loaded onto the capillary. By concentrating a large amount of sample at the beginning of the capillary, prior to separation, loadability is increased, and detection levels can be lowered several orders of magnitude. The most popular method of preconcentration in CE is sample stacking. Sample stacking has recently been reviewed (Chien and Burgi, Anal. Chem. 64:489A, 1992). Sample stacking depends of the matrix difference, (pH, ionic strength) between the sample buffer and the capillary buffer, so that the electric field across the sample zone is more than in the capillary region. In sample stacking, a large volume of sample in a low concentration buffer is introduced for preconcentration at the head of the capillary column. The capillary is filled with a buffer of the same composition, but at higher concentration. When the sample ions reach the capillary buffer and the lower electric field, they stack into a concentrated zone. Sample stacking has increased detectabilities 1 to 3 orders of magnitude.

Another method of preconcentration is to apply isotachophoresis (ITP) prior to the free zone CE separation of analytes. ITP is an electrophoretic technique which allows microliter volumes of sample to be loaded on to the capillary, in contrast to the low nL injection volumes typically associated with CE. The technique relies on inserting the sample between two buffers (leading and trailing electrolytes) of higher and lower mobility respectively, than the analyte. The technique is inherently a concentration technique, where the analytes concentrate into pure zones migrating with the same speed. The technique is currently less popular than the stacking methods described above because of the need for several choices of leading and trailing electrolytes, and the ability to separate only cationic or anionic species during a separation process.

Microfabricated Devices

Capillary electrophoresis (CE) is a powerful method for DNA sequencing, forensic analysis, PCR product analysis and restriction fragment sizing. CE is far faster than traditional slab PAGE since with capillary gels a far higher potential field can be applied. However, CE has the drawback of allowing only one sample to be processed per gel. The method combines the faster separations times of CE with the ability to analyze multiple samples in parallel. The underlying concept behind the use of microfabricated devices is the ability to increase the information density in electrophoresis by miniaturizing the lane dimension to about 100 micrometers. The electronics industry routinely uses microfabrication to make circuits with features of less than one micron in size. The current density of capillary arrays is limited the outside diameter of the capillary tube. Microfabrication of channels produces a higher density of arrays. Microfabrication also permits physical assemblies not possible with glass fibres and links the channels directly to other devices on a chip. Few devices have been constructed on microchips for separation technologies. A gas chromatograph (Terry et al., IEEE Trans. Electron Device, ED-26:1880, 1979) and a liquid chromatograph (Manz et al., Sens. Actuators B1:249, 1990) have been fabricated on silicon chips, but these devices have not been widely used. Several groups have reported separating fluorescent dyes and amino acids on microfabricated devices (Manz et al., J. Chromatography 593:253, 1992; Effenhauser et al., Anal. Chem. 65:2637, 1993). Few years ago Woolley and Mathies (Woolley and Mathies, Proc.

Natl. Acad. Sci. 91:11348, 1994) have shown that photolithography and chemical etching can be used to make large numbers of separation channels on glass substrates. The channels are filled with hydroxyethyl cellulose (HEC) separation matrices. It was shown that DNA restriction fragments could be separated in as little as two minutes.

Most preferred methods of separation are denaturing gradient gel electrophoresis (DGGE), and capillary electrophoresis, because these techniques allow for a separation of molecules presenting only a single base or aminoacid difference in their sequence.

**Detection of Polymorphism at step a), or at step a2)**

Preferably, in the method according to the invention, the polymorphism of nucleic acids and proteins is detected at step a).

Any approach available to analyse polymorphism in a sample can be used to perform step a). Preferred approaches, which make use of detection systems and separation substrates as defined above, are briefly disclosed hereunder :

- Single- Strand Conformation Polymorphism (SSCP)

This technique is a way of analyzing polymorphisms on the basis of conformational differences in denatured DNA. Briefly, restriction enzyme digestion or PCR is used to produce relatively small DNA fragments which are then denatured and resolved by electrophoresis on non-denaturing polyacrylamide gels. Conformational differences in the single-stranded DNA fragments resulting from base substitutions are detected by electrophoretic mobility shifts. Intra-strand base pairing creates single strand conformations that are highly sequence-specific and distinctive in electrophoretic mobility. The method can be used to analyze polymorphisms based on short insertions or deletions. This method is one of the most powerful tools for identifying point mutations and deletions in DNA (SSCP-PCR, Dean et al., Cell 61:863, 1990).

Accordingly, the method of the invention comprises a step of a) amplifying by polymerase chain reaction (PCR) nucleic acids molecules from a sample, before step b) consisting of submitting said amplified molecules to a separation step according to their size.

Preferably, the size separation step is non-denaturing and the nucleic acid fragments are denatured prior to the separation methodology. The size separation step can be accomplished, for example by gel electrophoresis (e.g., polyacrylamide gel electrophoresis), capillary electrophoresis or HPLC.

- RFLPs

Restriction endonucleases recognize short DNA sequences and cut DNA molecules at those specific sites. Some restriction enzymes (rare-cutters) cut DNA very infrequently, generating a small number of very large fragments (several thousand to a million bp). Most enzymes cut DNA more frequently, thus generating a large number of small fragments (less than a hundred to more than a thousand bp). On average, restriction enzymes with 4-base recognition sites will yield pieces 256 bases long, 6-base recognition sites will yield pieces 4000 bases long, and 8-base recognition sites will yield pieces 64,000 bases long. Since hundreds of different restriction enzymes have been characterized, DNA can be cut into many different small fragments.

A wide variety of techniques have been developed for the analysis of DNA polymorphisms. The most widely used method, the restriction fragment length polymorphism (RFPL) approach, combines restriction enzyme digestion, gel electrophoresis, blotting to a membrane and hybridization to a cloned DNA probe. Polymorphisms are detected as variations in the lengths of the labelled fragments on the blots. The RFLP approach can be used to analyze base substitutions when the sequence change falls within a restriction enzyme site or to analyze minisatellites/VNTRs by choosing restriction enzymes that cut outside the repeat units. The agarose gels do not usually afford the resolution necessary to distinguish minisatellite/VNTR alleles differing by a single repeat unit, but many of the minisatellites/VNTRs are so variable that highly informative markers can still be obtained.

**Preferred embodiments of the method according to the invention**

According to the invention, at step a), or in some embodiments at step a3), the surface area of migration is divided in a series of blocks along the X-axis, each block having the same surface. This series can be a discrete series of blocks, the number of which will depend on the method used to divide said surface. It is to be noted that step a), or in some embodiments step a3), is one of the key steps of the method according to the invention, because the working of the said step greatly influences the final accuracy of the method. Indeed, the accuracy of the method according to the invention will increase with the number of blocks along the X-axis.

As used herein, the term "localisation value" refers to the position, of a block, wherein the said block is located on the surface area of a separation substrate used according to the method of the invention. The localisation value of the said block is expressed by a location along the X-axis of the said separation substrate so as the blocks are ordered along the X-axis. The kind of measure units which are used to express the spot location on the X-axis is not an essential feature of the method. These units may be expressed as millimeters or centimeters, or even as a retention time in seconds, if an HPLC separation substrate or a capillary electrophoresis system is used. In the case wherein the surface area of the separation substrate is scanned so as to generate an electronic image of the migration area before determining the localisation value of each block, the X-axis co-ordinates of each block is preferably expressed as pixel co-ordinates, along X-axis.

Samples to analyse comprises molecules consisting of nucleic acids or proteins. The number of these molecules is quantified at the beginning of step b), and in some embodiments at step b1).

Any method known from the man skilled in the art can be used to quantify molecules consisting of nucleic acids or proteins.

Examples of these methods are disclosed below and mainly consists of the detection of a signal emitted by these molecules, whom intensity is quantified. This signal can be provided for example by a conventional protein or nucleic acid staining.

In order to enhance signal intensity, the said nucleic acids or protein can be labelled with a detectable molecule. The role of the detectable molecule consists of amplifying a detectable signal provided by every nucleic acid or protein included in the array.

Detection systems of nucleic acids commonly, and almost exclusively, utilize an intercalating dye or radioactive label, or, a nonradioactive label. Intercalating dyes, such as ethidium bromide, are simple to use. The dye is included in the gel matrix during electrophoresis or, following electrophoresis, the gel is soaked in a dye-containing solution. The dye can be directly visualized in some cases, but more often, and for ethidium bromide in particular, is excited by light (e.g., UV) to fluoresce. A more sensitive detection technique than dyes uses a radioactive (or nonradioactive) label. Typically, either a radiolabelled nucleotide or a radiolabelled primer is included during a PCR reaction. Following separation, the radiolabel is "visualized" by autoradiography. In such systems, nucleotides contain a label, such as a fluorophore, biotin or digoxin, which can be detected by an antibody or other molecule (e.g., other member of a ligand pair) that is labelled with an enzyme reactive with a chromogenic substrate. These systems do not have the safety concerns as described above, but use components that are often labile and may yield nonspecific reactions, resulting in high noise intensity (i.e., low signal-to-noise ratio).

In order to enhance signal intensity of nucleic acids, nucleic acids of the sample can also be amplified by a polymerase chain reaction (PCR) before migration.

Proteins for analysis can also be labelled prior to separation. In a typical embodiment proteins are radioactively marked by substituting certain amino acids with radioactively labelled amino acids. To those familiar with the art methionine labelled with [$^{35}$S] is frequently used. Other alternatives are to make use of biotinylated amino acids in which lateral side chains of the amino acid are covalently linked to a biotin molecule.

After separation, molecules can be detected by a number of techniques currently in use including (but not limited to) auto-radiographic exposure of light sensitive film, phosphorescene imaging such as with a Storm phosphor imager Molecular Dynamics electroimaging using an Instant Imager (Packard Instruments Inc.), according to the manufacturer's instructions. In a typical embodiment, the TLC plate is air dried after the electrophoretic and chromatographic steps to evaporate solvents and exposed directly in an Instant Imager (Packard Instruments Inc.) according to the manufacturers instructions (Instant Imager User's Guide No 7001158, Publication, pp. 169-4106).

Fluorescence detection can also be used in combination with capillary electrophoresis for samples that naturally fluoresce or are chemically modified to contain fluorescent tags This mode of detection offers high sensitivity and improved selectivity for these samples. Laser-induced fluorescence can also be used in capillary electrophoresis systems with detection limits as low as 10-18 to 10-21 mol. The sensitivity of the technique is attributed to the high intensity of the incident light and the ability to accurately focus the light on the capillary.

Preferred embodiments for performing steps (a) to (d) of the method, or in some embodiments steps (a3) to (d) of the method, through the use of one or several computer program(s).

As it has been already mentioned earlier in the present specification, a computer program, or alternatively a specific module or sub-program of a computer program, may be used to digitize the surface area of the separation substrate after migration of the sample molecules, and to generate a set of data which are representative of the digitised image of the said surface area, which set of data are then stored into a storage mean of the computer, e.g. a computer memory or a computer mass storage medium like a hard disk. In a preferred embodiment, the said computer program, or alternatively the said computer module or sub-program, performs as follows : the said first module, acquires image data derived from the sample molecules migration surface area, after that a separation of the molecules contained in the sample to study have been performed, e.g. by an electrophoresis separation method, and preferably by a capillary electrophoresis separation method. Then the corresponding image file comprising said image data is stored in the appropriate storage mean of the computer. The computer program advantageously contains instructions for monitoring the working of an image capture device, including for instance PhospholImager® (Molecular Dynamics Inc, Mountain View, CA) or Instant Imager®.

A second computer program, or alternatively a second program module or sub-program, may be used for performing further steps (a) to (c) of the method, or in some embodiments steps (a3) to (b5) of the method, *i.e.* representing on a graph, the intensity of a signal, corresponding to the presence of molecules from said sample on the migration surface area, as a function of distance from a point of origin on the migration area, to obtain a curve comprising peaks and valley, each peak corresponding to a maximum intensity of the signal for a location along the migration axis (X-axis), and each valley corresponding to a minimum intensity of the signal for a location along the said migration axis.

To achieve this goal, the set of data contained in the image file obtained with the computer program above is read by the second program or program module. The said digital image representation is divided in a series of blocks along the X-axis (i.e. the sample molecules migration axis), at step (a) of the method according to the invention, or in some embodiments at step (a3) of the method according to the invention. During this computer-implemented step, a location value is assigned for each block contained in the thus block-divided surface area of migration.

The magnitude or intensity of the signal that is emitted by molecules contained in each block, is quantified from the image dataset previously stored, to obtain an intensity value, the intensity value being representative of the number of molecules in each block.

A number of experimental ordered pairs equal to the number of blocks, is generated, each ordered pair having an X-coordinate and an Y-coordinate, and wherein :

(i) the location value of one block consists of the X-coordinate of one ordered pair, and
(ii) the intensity value of the signal corresponding to the block cited in (i), consists of the Y-coordinate of the ordered pair cited in (i).

Then, a two dimensional surface area defined by an X-axis and an Y-axis is created, and each ordered pair is represented as a point onto the two-dimensional surface area, using the X-coordinate and the Y-coordinate of each ordered pair for each point, respective to the X-axis and Y-axis,

Finally, each point is connected by a line to the one having the closest X-coordinate, to obtain a curve comprising a number (n) of peaks, and valleys.

All the steps above can be achieved for example by the software Matlab® (The Mathworks Inc., Natick, MA) or R (The R Foundation for Statistical Computing, Vienna, Austria).

A third program, or alternatively program module or sub-program, may be used for performing step (c) of the method, or in some embodiments steps (b5) to (c4), *i.e.* separating the peaks obtained above from a background. To achieve this goal, this program contains instructions for, respectively :

c1) determining the X-coordinate of each bottom on the two-dimensional surface area,
c2) connecting by a line each bottom to the one having the closest X-coordinate, to obtain a baseline,
c3) determining for each of the (n) peaks the surface area constituted by the contours of the peak and the baseline,
c4) assigning a number (n) of values $(a'_1, a'_2, ..., a'_n)$, value $a'_p$ being representative of the area of peak p.

For example a program using a valley to valley technique or a rolling ball technique can be used. All the above steps can be achieved for example by the software Matlab® (The Mathworks Inc., Natick, MA) or R (The R Foundation for Statistical Computing, Austria, Vienna).

A fourth program, or alternatively program module or sub-program, may be used for performing further step (c) and step (d) of the method, or in some embodiments steps (c5) to (d) of the method, in order to obtain a value representative of the biological diversity in the sample. To achieve this goal, this program contains instructions for, respectively :

c5) determining the surface area between the baseline and the X-axis from the lower limit to the upper limit, said surface consisting of a background,
c6) assigning a value (b') representative of the surface area between the base line and the X-axis, and
d) normalising the values $(a'_1, a'_2, ..., a'_n)$ and b' into the values $(a_1, a_2, ..., a_n)$ and b, using the formulas:

$$a_p = \frac{a'_p}{\sum\limits_{p=1}^{n} a'_p + b'} \quad \text{and} \quad b = \frac{b'}{\sum\limits_{p=1}^{n} a'_p + b'}$$

such that

$$\sum_{p=1}^{n} a_p + b = 1,$$

e) estimating the logarithmic Simpson diversity index (Z) of the said sample by calculating the estimated logarithmic Simpson diversity index ($Z_1$) by the formula:

$$Z_1 = -\ln \sum_{p=1}^{n} a_p^2 .$$

All the steps above can be achieved for example by the software Matlab® (The Mathworks Inc., Natick, MA) or R (The R Foundation for Statistical Computing, Vienna, Austria).

It has been found according to the invention that the experimental estimation of diversity of a sample, can be mathematically corrected through a correction factor (c) determined by applying the method of the invention to a set of computer-generated samples, whom diversity is known.

In a preferred embodiment of the method according to the invention the value $Z_1$ is corrected by a correcting factor (c).

Accordingly, the invention also concerns a method for measuring biological diversity in a sample, comprising the following steps :

A) providing a set of computer-generated samples, each of said computer-generated samples comprising a known number of species (S), and each of said species having a known relative abundance. The relative abundance of species (1, 2,..., S) is denoted by ($n_1$, $n_2$,..., $n_S$),

B) determining for each computer-generated sample the logarithmic Simpson diversity index Z, using the formula:

$$Z = -\ln \sum_{i=1}^{S} n_i^2 ,$$

C) determining for each computer-generated sample the value $Z_1$, by first generating a corresponding fingerprinting profile, and next applying step c) to step e) as defined in the method above,

D) calculating the correction factor (c) by performing a linear regression between the set of pairs (Z; $Z_1$) obtained in steps B) and C). The correction factor c satisfies:

$$Z \approx cZ_1,$$

E) providing a biological sample, comprising one or more molecules selected from nucleic acids and proteins, said molecules belonging to one or more species, and wherein molecules belonging to a same species have identical nucleotide or aminoacid sequence,

F) determining the value $Z_1$ of said sample according to step b) to step e) as defined in the method above,

G) calculating the value $Z_{1c}$ so as :

$$Z_{1c} = cZ_1,$$

wherein the value $Z_{1c}$ is the corrected estimated logarithmic Simpson diversity index and therefore a measure of the biological diversity of said sample.

It has been shown according to the invention that the diversity measurement obtained with the method according to the invention can be used as a tool for judicious community selection in metagenomic projects. These projects often rely on a blind choice of the microbial assemblage to be analysed.

The Simpson diversity index is directly related to the probability that two randomly drawn DNA sequences are different. The inventors have found that the Simpson diversity index also allows prediction of the relationship between the sequencing effort and the probability of discovering new DNA.

In example 4, a microbial community from which genetic sequences are randomly and repeatedly drawn has been considered, assuming a bacterial genome size of 3.2 million bp (Xu J et al.,) and a sequence run of 800 bp. During this sampling procedure, a rarefaction of newly discovered DNA occurs. $K_\alpha$ denotes the number of sequences analyzed before the expected quantity of redundant DNA rises to a fraction $\alpha$ of the total quantity of analyzed DNA (Figure 7). The Simpson diversity index Z is strongly correlated to the sequencing effort $K_\alpha$ for small $\alpha$, i.e., when the probability to retrieve new DNA remains high, $\ln K_\alpha \approx Z + z(\alpha)$, with $z(\alpha)$ being a constant independent of the composition of the microbial community (Figure 7).

In other words, the inventors have found that measuring biological diversity with the method according to the invention allows a prediction of the sequencing effort before the probability of drawing previously analyzed DNA becomes appreciable. (Example 4 below)

As an example, for the most diverse soil microbial community, the Simpson diversity index is estimated to be 8.4, corresponding to a sequencing effort of $K_{1\%} = 10^{5.6 \pm 0.2}$ and $K_{50\%} = 10^{7.3 \pm 0.4}$ sequences. The standard deviation takes into account both the estimation error for Z (0.2) and for $K_\alpha$ (0.01 for $\alpha$ = 1% and 0.4 for $\alpha$ = 50%). Converted to an amount of DNA sequenced, the equivalent of 100 bacterial genomes should be analyzed to obtain 1% of redundant DNA ($K_{1\%}$), and the sequencing effort to discover new DNA half of the time ($K_{50\%}$) is comparable to 10 times the well-known Sargasso sea metagenomic library (Venter et al.,). In example 1, it is mentioned that the bacterial diversity found in human faeces varies from 2.0 to 5.7 due to intersubject variability (Table 1). Comparing the two extreme samples in a metagenomic library, the same DNA rarefaction is obtained for sequencing efforts differing by a factor $e^{5.7-2.0} \approx 40$. In the approaches described in the state of the art, this information is only obtained after a huge number of sequencings. Now, according to the present method, fingerprinting the microbial community under investigation, estimating the logarithmic Simpson diversity index Z with the estimator $Z_1$, and computing the corresponding sequencing effort, provides an accurate access to the same information before any sequencing.

Accordingly, the diversity measurement obtained by the method according to the invention allows prediction of the sequencing effort before initiating a metagenomic survey and allows selection of the most appropriate community.

The invention is further illustrated by the following examples.

## Examples

### Example 1: Application of the method according to the invention to SSCP fingerprinting profiles of DNA from various environments

In Table I, below the results of application of the method of the invention to 669 SSCP fingerprinting profiles of DNA from various environments have been gathered.

The estimator $Z_1$ has been corrected by a correcting factor c = 0.93 to get the unbiased estimator $Z_{1c}$ (Figure 2). The standard deviation of the diversity estimation is 0.2. For comparison purposes, the estimator $Z_0$ mentioned above,

$$Z_0 = -\ln \sum_{p=1}^{n} a''^2_p \, ,$$

was calculated for soil samples and is indicated in italics.

The experimental results provide evidence for the discriminating power of the method of the invention for a large variety of ecosystems (Table 1). The experimental results provide evidence for the poor reliability of the estimator $Z_0$.

The method according to the invention, by calculating the estimator $Z_1$, reflects the diversity after DNA extraction and PCR amplification. The determined bacterial diversity ranged from 0.6 for enrichment cultures to 8.4 for soil. In comparison, the tree diversity of the tropical evergreen forest was estimated as $Z_1$ = 5.2 (Hubbell, He et al.,). The largest value of $Z_0$ for soil was 3.5 compared to 8.4 for $Z_1$, confirming the poor reliability of the estimator $Z_0$ (Table 1 and Figure 2). Diversity seems to be a characteristic of every category of biotope.

The classification of microbial communities in decreasing order of complexity is soil; compost and gut; anaerobic digester; faeces, waste water, and sea water; and root, milk, and drinking water. Our analyses strengthen the assertion that soils support much more diverse microbial communities than aquatic ecosystems (Curtis et al.,). The cultivable fractions of soil, root, and milk were always less diverse than the original communities (Table 1).

Moreover, the cultivation of soil microorganisms seems to affect diversity more drastically than for other ecosystems. This result is in accordance with observations of the lowest proportion of cultured bacteria in soil, expressed as the number of individuals (Amann et al.,) or as the number of species (Torsvik et al.,). Sub-categories with differing diversity levels are apparent. In human faeces, the method according to the invention allows the distinction of babies bacterial communities from those of adults. The wide diversity range for a given category allows the exploration of temporal variations or geographical origins of microbial communities in terms of diversity. For example, in human faeces, the estimated Simpson diversity varies from 2.0 to 5.7. These trends are in accordance with conclusions from previous studies in which significant intersubject variability was described in the dominant species (Eckburg et al., Mueller et al.,). The method according to the invention, leading to a new estimator provide a tool to study the effects of environmental change (nutrition, administered antibiotic) or predisposition to diseases (age, maladapted host immune response) on human-associated microbes.

**Table 1 : Results of application of the method of the invention to 669 SSCP fingerprinting profiles of DNA from various environments:**

| Origin of DNA source | | Number of experiments | Mean $Z_{1c}$ (+/- SD) | Range of $Z_{1c}$ values |
|---|---|---|---|---|
| Categories | Sub-categories | | | |
| Aquatic | Total | 70 | 3.3 (1.1) | 1.0-5.5 |
| | Sea water | 11 | 4.8 (0.6) | 4.0-5.5 |
| | Waste water | 12 | 4.4 (0.6) | 3.3-5.4 |
| | Drinking water | 47 | 2.8 (0.8) | 1.0-4.8 |
| Terrestrial | Total | 140 | 5.0 (2.0) | 1.0-8.4 |
| | Soil | 35 | 7.4 (0.6) | 6.0-8.4 |
| | *Soil* | *35* | *3.0 (0.2)* | *2.4-3.5* |
| | Compost | 35 | 6.2 (0.7) | 4.4-7.7 |
| | Root | 70 | 3.3 (1.1) | 1.0-5.4 |
| Anaerobic digesters | | 123 | 5.3 (0.8) | 3.4-7.3 |
| Gut | | 34 | 6.3(1.3) | 2.9-8.0 |
| Feces | Total | 90 | 4.5 (1.2) | 2.0-7.5 |
| | Ruminal | 9 | 5.9 (1.6) | 3.3-7.5 |
| | Human adult | 43 | 4.5 (1.2) | 3.2-5.7 |
| | Human baby | 15 | 3.2 (0.7) | 2.0-4.3 |
| | Others | 23 | 4.7 (1.3) | 2.6-6.8 |
| Milk | Total | 16 | 3.0 (0.9) | 1.7-4.4 |
| | Cow | 7 | 3.8 (0.4) | 3.3-4.4 |
| | Camel | 3 | 2.8 (0.3) | 2.6-3.1 |
| | Goat | 6 | 2.2 (0.6) | 1.7-3.3 |
| Divers | | | | |
| | Aerosol | 21 | 4.7(0.7) | 3.3-6.1 |
| | Autotrophic reactor | 43 | 3.7 (0.9) | 2.2-6.0 |
| Cultivable fraction of | | | | |
| | Soil | 65 | 2.2 (0.8) | 0.6-5.9 |
| | Root | 62 | 2.6 (0.7) | 1.0-3.9 |
| | Cow milk | 5 | 2.8(0.6) | 2.0-3.6 |
| Total | | 669 | 4.2(1.7) | 0.6-8.4 |

**Example 2: Single Strand Conformation Polymorphism (SSCP)**

A SSCP analysis allows to separate DNA fragments of the same size but with a different composition. It relies on the fact that a single nucleotide modification can change the conformation of a single-strand molecule. The use of fluorescent dye-labelled DNA permits a highly sensitive laser detection, and capillary electrophoresis provides an optimal molecules separation.

The DNA was analyzed by PCR amplification of the bacterial 16S rRNA gene V3 region as described in (Delbès et al.). Twenty-five cycles of amplification were performed with a Mastercycler ep thermocycler (Eppendorf). The reaction mixtures contained 1X polymerase buffer, 0.2 mM dNTPs, 0.4 mM each primer, 2.5 U Pfu Turbo DNA polymerase (Stratagene), 1 μl sample genomic DNA, and water to bring the total volume to 50 μl. If necessary, T4 gp32 (Qbiogene) was added to improve amplification efficiency in low purity DNA. The PCR conditions were as follows: the initial denaturation step for 2 min at 94°C followed by 25 cycles of a three-stage program with 30 s at 94 °C, 30 s at 61 °C, 30 s at 72°C, and a final elongation for 10 min at 72°C. The reactions were stopped by cooling the mixture to 4°C. Amplification product sizes were confirmed by electrophoresis on a 0.7 % (wt/vol) agarose gel.

One μl of diluted PCR products was added to 18 μl of template suppression reagent loading buffer and 1 μl of ten times diluted internal size standard Rox 400 HD (Applied Biosystems). The sample was then denatured for 5 min at 94°C and placed directly on ice for 5 min. SSCP was performed using the ABI 3130 genetic analyzer (Applied Biosystems) equipped with four 50 cm capillary tubes filled with 5.6 % of conformation analysis polymer (Applied Biosystems) in corresponding buffer and 10 % glycerol. The injection of DNA in capillaries required 5 kV during 3 s. Electrophoresis was carried out at 15 kV and 32°C for 30 min per sample.

**Example 3: Simulating SSCP profiles**

The approach to simulate SSCP fingerprint profiles consists two steps. First, a microbial community has been generated. For our purpose, a species is characterized by its mean migration position. A microbial community is then given by the number of species $S$ and, for every species $i$ = 1, 2,... $S$, its relative abundance $n_i$ and its mean migration position $m_i$. Next, a SSCP profile corresponding to this community has been constructed. This yields a function $f$, which associates with the migration position $x \in$ R the signal intensity $f(x) \geq 0$.

A SSCP profile has been modelled as a sum of Gaussian peaks. It has been assumed that every species contributes exactly one peak to the profile. The width of the peak is the same for all species, will be denoted by $\sigma_g$ and can be considered as a measure for the experimental resolution. The peak centre position and the peak height are different from species to species. For a species $i$, the peak centre position is taken equal to its mean migration position $m_i$, and the peak height proportional to its abundance $n_i$. The latter proportionality requires some explanation. In our experimental setup, the absolute signal intensity is not calibrated. Two SSCP profiles which only differ by a constant factor, are therefore considered identical. This allows for a normalization of the profile $f$, which, due to our modelling assumptions, corresponds to a constant multiplying the species abundances $n_s$. It can therefore be dealt with relative rather than absolute abundances. The generation of a SSCP profile $f$ for a microbial community ($S$; $n_i$; $m_i$) can then be summarized as

$$f(x) = c \sum_{i=1}^{S} n_i g(x, m_i, \sigma_g),$$

with $g(x; m; \sigma)$ a Gaussian peak with mean $m$ and width $\sigma$,

$$g(x, m, \sigma) = \frac{1}{\sqrt{2\pi}\sigma} \exp\left(-\frac{(x-m)^2}{2\sigma^2}\right).$$

With this definition and because the relative abundances $n_s$ sum up to one,

$$\int f(x)dx = c.$$

We put $c$ = 1, except in the figures where $c$ is taken such that max $f(x)$=1.

To constitute a microbial community, we randomly generate $(n_i; m_i)$ for all species $i$ =1, 2, ..., $S$. The mean migration positions $m_i$ are taken independently and from the same distribution law. It has been checked that the results do not depend on the probability density function for $m_i$, as long as this function varies slowly over the scale $\sigma_g$.

To obtain the relative abundances $n_i$, the absolute abundances $N_i$ have been first independently generated from the same distribution law. Subsequently, the abundances $N_i$ is divided by the total number of individuals $\Sigma_i N_i$. This normalisation typically allows to eliminate one parameter of the distribution. The common species abundance distributions (SADs) is considered, and it is focused in particular on those which have one effective parameter:

The lognormal distribution, i.e.,

$$P(N_i \in [N, N+dN]) = \frac{1}{\sqrt{2\pi}\sigma N} \exp\left(-\frac{(\ln N - \mu)^2}{2\sigma^2}\right) dN \quad \text{with} \quad N \in ]0, \infty[$$

The left-hand side of this equation should be read as the probability that $N_i$ belongs to $[N, N+dN]$. Dividing by the total number of individuals eliminates the parameter $\mu$. When generating randomly lognormal distributions, the parameter $\sigma$ is chosen uniformly in [0,4].

The so-called geometric distribution, i.e.,

$$P(N_i \in [N, N+dN]) = \frac{1}{aN} dN \quad \text{with} \quad N \in [1, \exp(a)]$$

The restriction to the interval $[1, \exp(a)]$ is necessary to get an integrable distribution. When generating randomly geometric distributions, the parameter $a$ is chosen uniformly in [0,20].

The power-law distribution, i.e.,

$$P(N_i \in [N, N+dN]) = \frac{z-1}{N^z} dN \quad \text{with} \quad N \in [1, \infty[$$

The distribution is integrable for $z > 1$. When generating randomly power-law distributions, the parameter $z$ is chosen uniformly in [1.2,3].

The inventors believe these three one-parameter distributions with the mentioned parameter ranges, to cover a broad range of realistic SADs. An estimation procedure that works equally well for these distributions, can therefore be considered to be independent of the species abundance distribution.

Figure 4 gives an overview of the profiles obtainable for the lognormal SAD. Indeed, it suffices to vary $S$ and $\sigma$, as changing the lognormal parameter $\mu$ corresponds to multiplying the profile with a constant factor. Notice that similar profiles can be obtained for different parameter combinations. This has important consequences for the estimation problem. Indeed, it is expected that a function of the abundances $ns$ which takes different values for communities with similar profiles, will be difficult to estimate accurately from the SSCP profiles. Analogous observations can be made for the geometric and power-law distribution.

Consider a community consisting of $S$ species with relative abundances $n_i$, $i$ = 1, 2, ..., $S$. Apart from the number of species $S$, following quantities are often used to quantify the diversity of the community:

$$\text{Shannon diversity index: } H = F\big((n_i)\big) = -\sum_{i=1}^{S} n_i \ln n_i$$

$$\text{Logarithmic Simpson diversity index: } D = G\big((n_i)\big) = -\ln \sum_{i=1}^{S} n_i^2$$

Figure 4 shows the isolines for the Simpson diversity index $Z$. It can be noted that similar patterns are roughly connected by such isolines. This suggests that SSCP profiles contain information about logarithmic Simpson diversity index $Z$ of the community, rather than, e.g., the number of species $S$. However, Fig 4 is somehow misleading: for $S$ and $\sigma$ given, the SSCP profile still contains randomness. As only one realization is plotted, the figure does not tell whether other realizations are similar. A better way to investigate which quantity is encoded in the SSCP profiles is shown in Figure 5. Communities were generated with ln $S$, $H$ or $Z$ close to some fixed value, and the corresponding SSCP profiles are plotted. Whereas profiles from communities with a fixed number of species $S$ show a lot of variation, this is less the case for a fixed Shannon diversity index $H$, and almost no variation is observed for a fixed logarithmic Simpson diversity index $Z$. Moreover, this similarity of the profiles remains valid for different SADs, see Fig. 1. This indicates that the index $Z$ is better encoded in the SSCP profile than the index $H$, and that both indices are better encoded than the number of species S. The diversity estimation are based on the peak areas in the SSCP profile. Suppose there are $n$ peaks, and denote their areas by $a_p$ for $p$ = 1, 2, ..., n. The area under the profile which cannot be attributed to a peak, i.e. the background, is denoted by $b$. Due to (1), we have

$$\sum_{p=1}^{n} a_p + b = 1,$$

To be compared with the normalization commonly used in literature (Fromin et al.,),

$$\sum_{p=1}^{n} a''_p = 1 \quad \text{with} \quad a''_p = \frac{a'_p}{\sum_{p=1}^{n} a'_p}.$$

The corresponding estimators are

$$H_0 = F\big((a''_p)\big) = -\sum_{p=1}^{n} a''_p \ln a''_p$$

$$Z_0 = G\big((a''_p)\big) = -\ln \sum_{p=1}^{n} a''^2_p.$$

The estimators used in the method according to the invention take the background $b$ into account. To this end, it has been considered, next to the $n$ peaks visible in the profile, $m$ additional peaks filling up the background. Define

$$a_m = (a_1, a_2, ..., a_n, \frac{b}{m}, \frac{b}{m}, ..., \frac{b}{m}),$$

where the fraction b/m is repeated m times. As this is a sequence of positive numbers summing up to one, it can be interpreted as a sequence of relative abundances. Although this new community $a_m$ will typically have a different structure than the original community ($n_i$), their diversity indices can be close.

Two extreme cases are distinguished. In the first one, it is assumed a huge number of species, all with a very small abundance, filling up the background. This corresponds to taking the limit $m \to \infty$. The following estimators are obtained : $H_1 = \lim_{m \to \infty} F(a_m) = \infty$

$$Z_1 = \lim_{m \to \infty} G(a_m) = -\ln \sum_{p=1}^{n} a_p^2$$

Obviously, $H_1$ is useless. The other case consists of filling up the background with a small number of abundant species. It is reasonable to assume that their abundance cannot exceed the smallest peak area $a_{min}$, as they would otherwise stick out of the background. This leads to $m = b/a_{min}$ and the estimators

$$H_2 = F(a_{b/a_{min}}) = -\sum_{p=1}^{n} a_p \ln a_p - b \ln a_{min}$$

$$Z_2 = G(a_{b/a_{min}}) = -\ln \left( \sum_{p=1}^{n} a_p^2 + b a_{min} \right)$$

From the construction of the estimators, it is expected that $Z_1$ overestimate, and $H_2$ and $Z_2$ underestimate the diversity.

Figure 6 compares the different estimators for the three SADs discussed earlier (lognormal, geometric and power-law). For each of them, 5000 communities were randomly generated, a corresponding SSCP profile was computed and analyzed, and the estimated diversities were compared to the diversity of the community. The estimators $Z_1$ and $Z_2$ have a smaller bias and a smaller variance than the estimator $H_2$. This is in accordance with the remark that Z is better encoded in the profile than H. Note also that the estimators $H_0$ and $Z_0$ are bounded from above by $\ln P$. This explains the saturation observed in the figure, as the number of peaks $P$ in a SSCP profile is typically smaller than 50 (Loisel et al.,).

The estimator $Z_1$ performs particularly well. In contrast with the other estimators, its curve does not exhibit any saturation, and is close to perfect linearity (Figure 6, see also Fig. 2). This constant relative bias, an overestimation of 8% in our simulations, weakly depends on the peak width $\sigma_g$, and thus on the experimental resolution. A trivial calibration yields an unbiased estimator, which was used on the experimental profiles (Figure 3).

## Example 4 Application: Metagenomics

Suppose we investigate a microbial community with S species and abundances $n_i$, $i$ = 1, 2, ..., S. Assume that the metagenome of this community is broken up in pieces, and that sequences of fixed length $N_{seq}$ are repeatedly analysed and compared to previously drawn sequences. Assume also that every species has a genome of the same length $N_{gen} = M N_{seq}$, consisting thus of M sequences. Typical values are $N_{gen}$ = 3:2 106 bp, $N_{seq}$ = 800 bp and $M$ = 4000.

During this sampling procedure, a rarefaction of newly discovered DNA occurs. The drawn sequences (say $K$) might overlap, and we have to distinguish between the quantity of analysed DNA (= $K N_{seq}$) and revealed DNA ($\leq K N_{seq}$). One can prove that after K draws the expected quantity $L(K)$ of revealed DNA is given by

$$L(K) = N_{gen} \sum_{i=1}^{S} 1 - \left( 1 - \frac{n_i}{M} \right)^K ,$$

where $n_s$ are relative abundances. The ratio of redundant DNA to analysed DNA is then

$$\ell(K) = 1 - \frac{L(K)}{KN_{seq}} = 1 - \frac{M}{K}\sum_{i=1}^{S}1 - \left(1 - \frac{n_i}{M}\right)^K$$

The function $\ell$ is monotonically increasing from 0 to 1. Therefore, for any $\alpha$ in $]0; 1[$, there is a corresponding $K_\alpha$ such that $\ell(K_\alpha) = \alpha$. Note that the solution $K_\alpha$ is not necessarily an integer. For sufficiently diverse communities, however, $K_\alpha \gg 1$ and a continuous approach is justified.

Figure 7 plots the sequencing effort $K_\alpha$ against the diversity indices $H$ and $Z$, for communities generated from the lognormal, geometric and power-law SAD. For each of them, 5000 communities were randomly generated, the numbers $K_\alpha$ for $\alpha = 0.01$, $\alpha = 0.1$ and $\alpha = 0.5$ were computed and compared to the diversity of the community. There is a strong correlation between the Shannon diversity index $H$ and the sequencing effort $\ln K_\alpha$ for $\alpha$ close to 0.5,
$\ln K_\alpha \approx H + h(\alpha)$, $\alpha \approx 0.5$.
The constant $h(\alpha)$ does not depend on the composition of the community. Similarly, there is a strong correlation between the logarithmic Simpson diversity index $Z$ and the sequencing effort $\ln K_\alpha$ for small $\alpha$,
$\ln K_\alpha \approx Z + z(\alpha)$, $\alpha \ll 1$.
Approximating the ratio of redundant DNA for large $M$ and small $n_i$ yields $z(\alpha) = \ln(2\alpha M)$, which fits the simulation results very well.

**References**

R. I. Amann, W. Ludwig, K. H. Schleifer, Microbiol. Rev. 59, 143 (1995).

T. P. Curtis, W. T. Sloan, J. Scannell, Proc. Natl. Acad. Sci. U.S.A. 99, 10494 (2002).

C. Delbès, R. Moletta, J.J. Godon, Env. Microbiol. 2, 506 (2000).

J. Dunbar, S. M. Barns, L. O. Ticknor, C. R. Kuske, Appl. Environ. Microbiol. 68, 3035 (2002).

P. B. Eckburg et al., Science 308, 1635 (2005).

N. Fromin et al., Env. Microbiol. 4, 634 (2002).

J. Gans, M. Wolinsky, J. Dunbar, Science 309, 1387 (2005).

D. Gevers et al., Nat. Rev. Microbiol. 3, 733 (2005).

F. He, X.-S. Hu, Ecol. Lett. 8, 386 (2005).

S.-H. Hong, J. Bunge, S.-O. Jeon, S. S. Epstein, Proc. Natl. Acad. Sci. U.S.A. 103, 117 (2006).

S. P. Hubbell, The Unified Neutral Theory of Biodiversity and Biogeography (Princeton Univ. Press, Princeton, 2001).

P. Loisel et al., Env. Microbiol. 8, 720 (2006).

S. Mueller et al., Appl. Env. Microbiol. 72, 1027 (2006).

N. R. Pace, Science 276, 734 (1997).

C. Pedros-Alio, Trends Microbiol. 14, 257 (2006).

C. E. Shannon, Bell Syst. Tech. J. 27, 379 and 623 (1948).

E. H. Simpson, Nature 163, 688 (1949).

V. Torsvik, F. L. Daae, R.-A. Sandaa, L. Øvreås, J. Biotechnol. 64, 53 (1998).

J. C. Venter, Science 304, 66 (2004).

J. Xu, Mol. Ecol. 15, 1713 (2006).

**Claims**

1. A computer-implemented method for measuring biological diversity in a sample comprising the following steps :

    a) submitting a sample to migration on the surface area of a migration substrate, wherein the said sample comprises one or more molecules selected from the group consisting of nucleic acids and proteins, wherein the said molecules belong to one or more species, and wherein molecules which belong to the same species have an identical nucleotide or aminoacid sequence;
    b) generating a fingerprinting profile from the migration pattern of the molecules on the surface area of the said migration substrate, the said fingerprinting profile comprising a number (n) of peaks ($P_1$, $P_2$, ..., $P_n$) and a background (B);
    c) from the fingerprinting profile generated at step b), determining respectively :

(i) the surface area (a'$_1$, a'$_2$, ..., a'$_n$) for each peak (P$_1$, P$_2$, ..., P$_n$) contained in the said fingerprinting profile;
(ii) the surface area (b') of the background (B);

d) normalising the values (a'$_1$, a'$_2$, ..., a'$_n$) and b' into the values (a$_1$, a$_2$, ..., a$_n$) using the formulas:

$$a_p \square \frac{a'_p}{\sum\limits_{p\square 1}^{n} a'_p \square b'} \quad \text{and} \quad b \square \frac{b'}{\sum\limits_{p\square 1}^{n} a'_p \square b'}$$

such that

$$\sum\limits_{p\square 1}^{n} a_p \square b \square 1.$$

e) estimating the logarithmic Simpson diversity index (Z) of the said sample by calculating the estimated logarithmic Simpson diversity index (Z$_1$) by the formula:

$$Z_1 \square \square \ln \sum\limits_{p\square 1}^{n} a'^2_p,$$

where the value Z$_1$ is a measure of the biological diversity in the sample.

2. The computer-implemented method according to claim 1, wherein step

a) comprises the following steps :

a1) providing a sample comprising one or more molecules selected from nucleic acids and proteins, said molecules belonging to one or more species, and wherein molecules belonging to a same species have identical nucleotide or aminoacid sequence ,
a2) submitting said sample to a separation step on the surface area of a separation substrate, whereby molecules migrate on the surface area, along a X-axis at different locations according to their nucleotide or aminoacid sequence,
a3) dividing the surface area in a series of blocks, along the said X-axis, each block having the same surface, and
a4) assigning a location value for each block contained in the surface area, wherein the location value consists of the location of each block, along the said X-axis, respective to a unique predetermined point of origin in the surface area, so as the blocks are ordered along the X axis.

3. The computer-implemented method according to any one of claims 1 and 2, wherein step b) comprises the following steps :

b1) quantifying the number of molecules at each block, to obtain an intensity value, wherein the intensity value is representative of the number of molecules in each block,
b2) generating a number of experimental ordered pairs equal to the number of blocks, each ordered pair having an X- coordinate and an Y-coordinate, and wherein :

(i) the location value of one block constitutes the X-coordinate of one ordered pair, and
(ii) the intensity value of the signal corresponding to the block cited in (i), constitutes the Y-coordinate of the ordered pair cited in (i),

b3) generating a two dimensional surface area defined by an X-axis and an Y-axis,
b4) representing each ordered pair as a point onto the two-dimensional surface area, using the X-coordinate and the Y-coordinate of each ordered pair for each point, respective to the X-axis and Y-axis, and
b5) connecting by a line each point to the one having the closest X-coordinate, to obtain a curve comprising a number (n) of peaks, and wherein :

> (i) each peak is separated from another peak by a valley,
> (ii) each peak comprises a top,
> (iii) each valley comprises a bottom,

4. The computer-implemented method according to any one of claims 1 to 3, wherein step c) comprises the following steps :

> c1) determining the X-coordinate of each bottom on the two-dimensional surface area,
> c2) connecting by a line each bottom to the one having the closest X-coordinate, to obtain a baseline,
> c3) determining for each of the (n) peaks the surface area delimited by the contours of the peak and the baseline,
> m) assigning a number (n) of values ($a'_1$, $a'_2$, ..., $a'_n$), value $a'_p$ being representative of the area of peak p,
> c4) determining the surface area between the baseline and the X-axis from the lower limit to the upper limit, said surface consisting of a background, and
> c5) assigning a value (b') representative of the surface area between the base line and the X-axis.

5. The computer-implemented method according to any one of claims 1 to 4 wherein the nucleic acids or proteins, are labelled with a detectable molecule.

6. The computer-implemented method according to any one of claims 1 to 5, wherein the nucleic acids are ribosomal RNA molecules.

7. A method according to any one of claims 1 to 6, wherein at step a) or a2), the said sample is submitted to a separation step selected from the group consisting of an electrophoresis separation step, or a chromatography separation step.

8. The computer-implemented method according to any one of claims claim 4 to 7, wherein the baseline is identified with a technique selected in the group consisting of a valley to valley technique or a rolling ball technique.

9. A method according to any one of claims 1 to 8, wherein, at step a), the molecules consist of nucleic acids and wherein the said nucleic acids are amplified previously to performing the separation step.

10. The computer-implemented method according to claim 9, wherein the said nucleic acid molecules from bacterial 16s rRNA gene V3 region.

11. A method for measuring biological diversity in a sample, comprising the following steps :

> A) providing a set of computer-generated samples, each of said computer-generated samples comprising a known number of species (S), and each said species having a known relative abundance. The relative abundance of species (1, 2,..., S) is denoted ($n_1$, $n_2$,..., $n_S$),
> B) determining for each computer-generated sample the logarithmic Simpson diversity index (Z), using the formula:

$$Z = \ln \sum_{i=1}^{S} n_i^2 \, ,$$

> C) determining for each computer-generated sample the value $Z_1$, by first generating a corresponding fingerprinting profile, and next applying step c) to step e) as defined in claim 1,
> D) calculating the correction factor (c) by performing a linear regression between the set of pairs ($Z$; $Z_1$) obtained in steps B) and C). The correction factor c satisfies
> $Z = cZ_1$,
> E) providing a biological sample, comprising one or more molecules selected from nucleic acids and proteins, said molecules belonging to one or more species, and wherein molecules belonging to a same species have identical nucleotide or aminoacid sequence,

F) determining the value $Z_1$ of said sample according to step b) to step e) as defined in claim 1,
G) calculating the value $Z_{1c}$ so as

$$Z_{1c} = cZ_1,$$

wherein the value $Z_{1c}$ is the corrected estimated logarithmic Simpson diversity index and therefore a measure of the biological diversity of said sample.

FIGURE 1

FIGURE 2

FIGURE 3

FIGURE 4

**FIGURE 5**

**A**

**B**

FIGURE 6

FIGURE 7

**European Patent Office**

**EUROPEAN SEARCH REPORT**

Application Number

EP 07 30 1485

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,A | LOISEL PATRICE ET AL: "Denaturing gradient electrophoresis (DGE) and single-strand conformation polymorphism (SSCP) molecular fingerprintings revisited by simulation and used as a tool to measure microbial diversity." ENVIRONMENTAL MICROBIOLOGY APR 2006, vol. 8, no. 4, April 2006 (2006-04), pages 720-731, XP002469878 ISSN: 1462-2912 * the whole document * in particular page 724, col. 2, paragraph 2 ----- | 1-11 | INV. G01N33/569 G01N30/86 |
| A | GORELICK ROOT: "Combining richness and abundance into a single diversity index using matrix analogues of Shannon's and Simpson's indices" ECOGRAPHY, vol. 29, no. 4, August 2006 (2006-08), pages 525-530, XP002469879 ISSN: 0906-7590 * the whole document * in particular page 526, col. 1, first full paragraph. ----- | 1-11 | |
| A | DUMESTRE JEAN-FRANCOIS ET AL: "Changes in bacterial and archaeal assemblages in an equatorial river induced by the water eutrophication of Petit Saut dam reservoir (French Guiana)" AQUATIC MICROBIAL ECOLOGY, vol. 26, no. 3, 18 January 2002 (2002-01-18), pages 209-221, XP002469880 ISSN: 0948-3055 * the whole document * in particular page 212, passage bridging cols. 1 and 2 ----- | 1-11 | TECHNICAL FIELDS SEARCHED (IPC) G01N G06F G06T |

The present search report has been drawn up for all claims

| Place of search | Date of completion of the search | Examiner |
|---|---|---|
| Munich | 21 February 2008 | Thumb, Werner |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HUBER et al.** *Anal. Biochem.,* 1993, vol. 212, 351
- **HUBER et al.** *Nuc. Acids Res.,* 1993, vol. 21, 1061
- **HUBER et al.** *Biotechniques,* 1993, vol. 16, 898
- **OEFNER et al.** *Anal. Biochem.,* 1994, vol. 223, 39
- **OLIVARES et al.** *Anal. Chem.,* 1987, vol. 59, 1230
- **SMITH et al.** *Anal. Chem.,* 1988, vol. 60, 436
- **LOO et al.** *Anal. Chem.,* 1989, vol. 179, 404
- **EDMONDS et al.** *J. Chroma.,* 1989, vol. 474, 21
- **LOO et al.** *J. Microcolumn Sep.,* 1989, vol. 1, 223
- **LEE et al.** *J. Chromatog.,* 1988, vol. 458, 313
- **SMITH et al.** *J. Chromatog,* 1989, vol. 480, 211
- **GRESE et al.** *J. Am. Chem. Soc.,* 1989, vol. 111, 2835
- **COHEN et al.** *Proc. Natl. Acad. Sci., USA,* 1988, vol. 85, 9660
- **SMITH et al.** *Nuc. Acids. Res.,* 1990, vol. 18, 4417
- **MATHIES ; HUANG.** *Nature,* 1992, vol. 359, 167
- **HUANG et al.** *Anal. Chem.,* 1992, vol. 64, 967
- **HUANG et al.** *Anal. Chem.,* 1992, vol. 64, 2149
- **CHIEN ; BURGI.** *Anal. Chem.,* 1992, vol. 64, 489A
- **TERRY et al.** *IEEE Trans. Electron Device,* 1979, vol. ED-26, 1880
- **MANZ et al.** *Sens. Actuators,* 1990, vol. B1, 249
- **MANZ et al.** *J. Chromatography,* 1992, vol. 593, 253
- **EFFENHAUSER et al.** *Anal. Chem.,* 1993, vol. 65, 2637
- **WOOLLEY ; MATHIES.** *Proc. Natl. Acad. Sci.,* vol. 91, 11348

- **DEAN et al.** *Cell,* 1990, vol. 61, 863
- Instant Imager User's Guide No 7001158. 169-4106
- **R. I. AMANN ; W. LUDWIG ; K. H. SCHLEIFER.** *Microbiol. Rev.,* 1995, vol. 59, 143
- **T. P. CURTIS ; W. T. SLOAN ; J. SCANNELL.** *Proc. Natl. Acad. Sci. U.S.A.,* 2002, vol. 99, 10494
- **C. DELBÈS ; R. MOLETTA ; J.J. GODON.** *Env. Microbiol.,* 2000, vol. 2, 506
- **J. DUNBAR ; S. M. BARNS ; L. O. TICKNOR ; C. R. KUSKE.** *Appl. Environ. Microbiol.,* 2002, vol. 68, 3035
- **P. B. ECKBURG et al.** *Science,* 2005, vol. 308, 1635
- **N. FROMIN et al.** *Env. Microbiol.,* 2002, vol. 4, 634
- **J. GANS ; M. WOLINSKY ; J. DUNBAR.** *Science,* 2005, vol. 309, 1387
- **D. GEVERS et al.** *Nat. Rev. Microbiol.,* 2005, vol. 3, 733
- **F. HE ; X.-S. HU.** *Ecol. Lett.,* 2005, vol. 8, 386
- **S.-H. HONG ; J. BUNGE ; S.-O. JEON ; S. S. EP-STEIN.** *Proc. Natl. Acad. Sci. U.S.A.,* 2006, vol. 103, 117
- **S. P. HUBBELL.** The Unified Neutral Theory of Biodiversity and Biogeography. Princeton Univ. Press, 2001
- **P. LOISEL et al.** *Env. Microbiol.,* 2006, vol. 8, 720
- **S. MUELLER et al.** *Appl. Env. Microbiol.,* 2006, vol. 72, 1027
- **N. R. PACE.** *Science,* 1997, vol. 276, 734
- **C. PEDROS-ALIO.** *Trends Microbiol.,* 2006, vol. 14, 257
- **C. E. SHANNON.** *Bell Syst. Tech. J.,* 1948, vol. 27, 379, 623
- **E. H. SIMPSON.** *Nature,* 1949, vol. 163, 688
- **V. TORSVIK ; F. L. DAAE ; R.-A. SANDAA ; L. ØVREÅS.** *J. Biotechnol.,* 1998, vol. 64, 53
- **J. C. VENTER.** *Science,* 2004, vol. 304, 66
- **J. XU.** *Mol. Ecol.,* 2006, vol. 15, 1713